# EUROPEAN PATENT SPECIFICATION

(11) **EP 3 978 909 B1**
(45) Date of publication and mention of the grant of the patent: **19.11.2025**
(21) Application number: 20199309.4
(22) Date of filing: 30.09.2020
(51) Int. Cl.: G01N 21/84

(54) **METHOD OF DETERMINING THE CONCENTRATION OF AT LEAST ONE ANALYTE IN A BODILY FLUID**
VERFAHREN ZUR BESTIMMUNG DER KONZENTRATION VON MINDESTENS EINEM ANALYTEN IN EINER KÖRPERFLÜSSIGKEIT
PROCÉDÉ DE DÉTERMINATION DE LA CONCENTRATION D'AU MOINS UN ANALYTE DANS UN FLUIDE CORPOREL

(43) Date of publication of application: 06.04.2022
(73) Proprietor: Roche Diabetes Care GmbH, 68305 Mannheim (DE); F. Hoffmann-La Roche AG, 4070 Basel (CH)
(72) Inventor: Berg, Max, 68305 Mannheim (DE); Alperowitz, Lukas, 80805 München (DE)
(74) Representative: Altmann Stößel Dick Patentanwälte PartG mbB

(56) References cited:
- WO-A1-2020/094594
- US-A1- 2018 143 179

## Description

### Technical Field

The present invention refers to a method of determining the concentration of at least one analyte in a bodily fluid. The invention further relates to a mobile device having at least one camera, to a kit comprising said mobile device and to a computer program and a computer-readable storage medium. The method, the mobile device, the kit, the computer program and the computer-readable storage medium may be used specifically in medical diagnostics, in order to for example quantitatively or qualitatively detect one or more analytes in one or more body fluids, such as for detecting glucose in blood and/or interstitial fluid. Other fields of application of the present invention, however, are feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers or other mobile devices has become more and more popular over the recent years. As an example, WO 2012/131386 A1 discloses a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample.

As opposed to laboratory measurements and measurements performed by using dedicated analytical measurement devices, when using mobile computing devices such as smart phones, various influences need to be taken into account. As an example, lighting conditions, positioning, vibrations or other more or less uncontrollable conditions are to be considered. Generally, procedures may be used which comprise determining these influences under laboratory conditions.

For example, US 9,185,764 B2 discloses a driver arrangement comprising a current regulator, to which at least one light-emitting diode can be connected, and a driver circuit that is coupled to the current regulator and features a measurement input for receiving a measurement signal that is dependent on the operation of the at least one light-emitting diode. The driver circuit is designed for adjusting the current level of the current regulator current of the current regulator in dependence on the measurement signal such that a light quantity emitted by the at least one light emitting diode has a constant brightness value.

CN 105744067 A describes a mobile terminal flashlight brightness adjustment method, comprising the steps: when the flashlight of a mobile terminal is opened, judging that whether the flashlight is in an automatic brightness adjusting mode; if the flashlight is in the automatic brightness adjusting mode, acquiring the brightness of a current environment where the mobile terminal is located; acquiring a flashlight driving current preset value corresponding to the brightness of the current environment; and adjusting the driving current of the flashlight to be the same as the driving current preset value, and driving the flashlight to adjust the illuminating brightness. In addition, a mobile terminal flashlight adjustment device and a mobile terminal is described therein. According to the mobile terminal flashlight brightness adjustment method, the flashlight can be automatically adjusted according to the brightness of the environment.

US 10,219,709 B2 discloses an optical proximity sensor assembly including an optical proximity sensor with an IR LED emitting light having an infrared wavelength, an IR photo detector sensitive to the infrared wavelength, an optical barrier blocking direct light rays from the LED to the IR photo detector and permitting reflected light rays to reach the at least one photo detector; and an electronic integrated circuit with an amplifier for amplifying a signal detected by the photo detector, an analog to digital converter, LED drivers, noise reduction and ambient light cancellation circuitry, and a digital interface for communication with a microcontroller. The optical proximity sensor is accommodated on a wearable carrier. A single sensor may include a plurality of identical or different LEDs, a plurality of photodiodes, or both. Also, several sensors may be placed on a person's skin along a vascular path to obtain data relating to blood flow and artery stiffness.

US 9,241,663 B2 describes a system and method for analysis of colorimetric test strip strips and disease management. The system can include an accessory that is operably coupled to a mobile device, the mobile device acquiring and/or analyzing images of the colorimetric test strips. The light box accessory can be detachably attached to the mobile device, or made to remain attached to the mobile device, but with the capability of having the light box accessory removed from the field of view of the camera for general photography purposes. In other embodiments, an image containing known calibration color(s) and reagent area(s) is obtained sans the light box for comparison with a previous calibration image to model changes in ambient lighting conditions and determine a color correction function. The correction can be applied to the detected reagent area color(s) for matching between the detected reagent area color(s) and reference color(s) on the reference chart. Optionally, the information can be processed and displayed to provide feedback, as well as transmitted to a health provider for analysis.

US 2017/0219569 A1 discloses a method for a mobile computing device with a camera and a screen to measure a test strip in a test strip module including, in a calibration phase before a sample is introduced to a reaction area on the test strip, detecting with the camera an ambient light effect when the test strip module is mounted on the face of the mobile computing device. In a measurement phase after the sample is introduced to the reaction area on the test strip, the method also includes illuminating the test strip with a light provided by a light source area on the screen, detecting with the camera one or more colors of the reaction area, correcting the detected one or more colors for the ambient light effect, correlating the corrected one or more colors to an analyte property, and displaying a value of the analyte property on the screen.

US 9,506,855 B2 describes a method, system and computer program for analyzing a colorimetric assay that includes obtaining an image of the assay, optionally correcting for ambient lighting conditions in the image, converting the intensity data for at least one of the red channel, the green channel, or the blue channel to a first data point, recalling a predetermined standardized curve, comparing the first data point with the standardized curve, and identifying the value for the assay parameter from the standardized curve.

US 10,299,674 B2 discloses mobile computer devices, front mounted optical systems and computer program products allowing perimetry measurement.

WO 2020/094594 A1 describes a method of performing an analytical measurement based on a color formation reaction in an optical test strip by using a mobile device having a camera as well as a computer program including computer-executable instructions for performing the method, and, further, a mobile device and a kit for performing an analytical measurement. The method comprises step a): providing an optical test strip having a test field without having a sample applied thereto; step b): capturing at least one first image of at least part of the test field of the optical test strip without having a sample applied thereto by using the camera with at least one image acquisition setting of the camera, specifically with a set of acquisition settings of the camera; step c): applying a sample, specifically a drop, of bodily fluid to the test field of the optical test strip; step d): waiting for a predetermined minimum amount of time; step e): capturing at least one second image of at least part of the test field of the optical test strip having the sample of bodily fluid applied thereto by using the camera with the one or more image acquisition settings of the camera, wherein the image acquisition settings of the camera are the same image acquisition settings of the camera as used in step b); and step f): determining an analytical measurement result value by using the first and the second image of the optical test field of the optical test strip, specifically by comparing the at least two images.

US 2018/0143179 A1 discloses a mobile device configured to detect sickle cell traits in a blood sample. The device comprises a mobile device with a camera operatively coupled to a microscope lens. An image converter is configured to receive an image from the camera and to perform a noise reduction procedure. The noise reduction procedure manipulates the image to a monochrome image and applies a Gaussian filter. A contour detector detects the contours of the image. An image analysis tool is configured to analyze the contours to identify discrete blood cells and clustered blood cells. The user is then notified if sickle cell traits are present based at least on the shape of the discrete blood cells.

Despite the advantages achieved by the methods and devices known in the art, several technical challenges remain. Specifically, the methods known in the art generally require capturing images in a manual mode of the mobile computing device. In case the ambient light is low, an automatic detection of a test element may not be possible. Thus, the automatic detection of the test element may be turned off. The check of the ambient light situation may lead to incorrect result when the user of the mobile computing device does not accurately align the mobile computing device with the test element. A further check of the ambient light after the measurement may detect an inadmissible ambient light situation. However, this may be determined only after the measurement and the application of blood onto the test element. Further, the settings of the camera of the mobile computing device in an automatic mode are generally unknown. Thus, checking the ambient light situation with a camera of the mobile computing device is generally not possible with the methods and devices known in the art.

### Problem to be solved

It is therefore desirable to provide methods and devices which at least partially address the above-mentioned technical challenges. Specifically, it is desirable to provide methods and devices which allow for a user-friendly mobile-based determination of a concentration of at least one analyte in a bodily fluid and which take the ambient light situation into account for the determination.

### Summary

This problem is addressed by a method of determining the concentration of at least one analyte in a bodily fluid, by a mobile device and a kit, by a computer program and a computer-readable storage medium with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a method of determining the concentration of at least one analyte in a bodily fluid by using a mobile device is disclosed. The mobile device comprises at least one camera and at least one ambient light detector. Therein, the ambient light detector comprises at least one of an ambient light sensor and an additional camera.

The method comprises the following steps. Specifically, step i. as described in the following may at least once be performed before step ii. is performed. Thus, steps i. and ii. specifically may be implemented as separate method steps, with step i. being performed at least once before step ii. is performed. Further, it is also possible to perform one or more of the method steps once or repeatedly. The method may comprise further method steps which are not listed.

The method comprises:
i. performing at least one ambient light check, the ambient light check comprising:
   i.1 using the ambient light detector for determining at least one item of ambient light information; and
   i.2 determining if at least one validity criterion on the item of ambient light information is fulfilled;
ii. if the validity criterion is fulfilled, determining the concentration of the analyte in the bodily fluid by evaluating at least one image of at least a part of at least one reagent test region of an optical test strip having a sample of the bodily fluid applied thereto, the image being captured by the camera of the mobile device.

The term "determining the concentration of at least one analyte in a bodily fluid", also referred to as an "analytical measurement", as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample or aliquot of bodily fluid. For example, the bodily fluid may comprise one or more of blood, interstitial fluid, urine, saliva or other types of body fluids. The result of the determining of the concentration, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value may be determined by the analytical measurement.

Consequently, the term "analyte concentration value", often also referred to as "analytical measurement result value", as used herein, is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value.

As outlined above, the method comprises using a mobile device, the mobile device having at least one camera and at least one ambient light detector. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. A larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The term "ambient light detector" as used herein is a broad term and is to be given its ordinary and customary meaning to a person of ordinary skill in the art and is not to be limited to a special or customized meaning. The term specifically may refer, without limitation, to a device comprising at least one element being sensitive to light and/or other electromagnetic radiation. The ambient light detector specifically may be configured for generating at least one item of information indicative of ambient light, such as a measurement signal and/or a digital value indicative of a brightness and/or illuminance of ambient light. The ambient light detector may directly or indirectly be coupled to a processor of the mobile device and may be configured for providing the item of information to the processor. The ambient light detector specifically may be separate from the camera of the mobile device. Thus, as outlined in further detail below, the ambient light sensor and the camera may be located on opposing sides of the mobile device. As an example, the ambient light sensor may be located on a front side of the mobile device facing the user, the front side having e.g. a display and/or one or more operating elements, and the camera may be located on a back side of the mobile device, e.g. a side facing an object such as the optical test strip.

The ambient light detector, as outlined above, is configured for sensing ambient light. The term "ambient light" as used herein is a broad term and is to be given its ordinary and customary meaning to a person of ordinary skill in the art and is not to be limited to a special or customized meaning. The term specifically may refer, without limitation, to light being present in an environment of the mobile device. Specifically, ambient light may refer to light being ambient to one or more of the mobile device, an optical test strip or a color reference card. The ambient light may be the result of a superposition of light arising from any natural source, such as sun light, and/or from any artificial source, such as artificial lighting. The ambient light detector may be configured for converting ambient light into an electronic signal, e.g. an analogue and/or a digital electronic signal, such as a voltage signal and/or an electric current signal, indicating qualitatively and/or quantitatively an intensity of the ambient light. The ambient light detector may specifically be configured for detecting the intensity of ambient light.

As outlined above, the ambient light detector comprises at least one of an ambient light sensor and an additional camera. Thus, the entity referred to as the "ambient light detector" either contains at least one light-sensitive element referred to as an "ambient light sensor" or at least one additional camera being separate from the camera mentioned above, or both.

Therein, the term "ambient light sensor" as used herein is a broad term and is to be given its ordinary and customary meaning to a person of ordinary skill in the art and is not to be limited to a special or customized meaning. The term specifically may refer, without limitation, to a device having at least one photosensitive element configured for detecting light and/or other electromagnetic radiation. Specifically, the photosensitive element of the ambient light sensor may be configured for detecting light in a visible spectral range and/or in an infrared spectral range. The visible spectral range may refer to a wavelength interval of light having a wavelength of 380 nm to 760 nm. The infrared spectral range may refer to a wavelength interval of light having a wavelength of 760 nm to 1 mm. For example, the photosensitive element may comprise at least one interface of semiconductor materials, such as a p-n-junction. The photosensitive element may specifically comprise at least one of a photodiode, a phototransistor, a photoconductor, a semiconductor element, specifically a photosensitive semiconductor element. The ambient light sensor may form part of the mobile device. Specifically, the ambient light sensor may be comprised by one or more components of the mobile device. For example, the ambient light sensor may be comprised by at least one display of the mobile device. Thus, many displays contain ambient light sensors for adapting the brightness of the display to the ambient lighting conditions. The ambient light sensor of the display, specifically on the front side of the mobile device, may be used in a dual-use also for performing step i.1. Additionally or alternatively, a further ambient light sensor may be integrated into the mobile device, such as separate from the display. The ambient light sensor and the camera, as outlined above, specifically may be located on opposing sides of the mobile device.

Additionally or alternatively, the ambient light detector may be or may comprise at least one additional camera. The term "additional camera" as used herein is a broad term and is to be given its ordinary and customary meaning to a person of ordinary skill in the art and is not to be limited to a special or customized meaning. The term specifically may refer, without limitation, to a further device according to the above-mentioned term "camera". Thus, in this option, the mobile device comprises at least two cameras, wherein at least one of the cameras, herein simply referred to as "the camera", is used in step ii., and wherein at least another camera, herein referred to as "the additional camera" is used for the ambient light check in step i.1. Specifically, the additional camera may be a camera device being physically separated from the camera of the mobile device. For example, the camera of the mobile device may be or may comprise a main camera of the mobile device, whereas the additional camera may be or may comprise a front camera of the mobile device. The camera and the additional camera may be located on opposing sides of the mobile device. For example, the additional camera, specifically the front camera may be arranged on a front side of the mobile device. The term "front side" as used herein is a broad term and is to be given its ordinary and customary meaning to a person of ordinary skill in the art and is not to be limited to a special or customized meaning. The term specifically may refer, without limitation, to a side of the mobile device facing a user of the mobile device. Specifically, the front side of the mobile device may comprise at least one operation element of the mobile device, specifically the at least one display and/or at least one touchscreen. The camera of the mobile device, such as the main camera, may be arranged at the back side of the mobile device. The term "back side" as used herein is a broad term and is to be given its ordinary and customary meaning to a person of ordinary skill in the art and is not to be limited to a special or customized meaning. The term specifically may refer, without limitation, to a side of the mobile device being opposite to the front side. Specifically, the back side of the mobile device faces one or more of an optical test strip or a color reference card when at least one image of at least one part of at least one reagent test region is captured.

As outlined above, the method comprises in step i., performing at least one ambient light check. The term "ambient light check" as used herein is a broad term and is to be given its ordinary and customary meaning to a person of ordinary skill in the art and is not to be limited to a special or customized meaning. The term specifically may refer, without limitation, to the process of a qualitative and/or quantitative evaluation of the ambient light as defined above. Specifically, the ambient light check may comprise quantifying the ambient light and checking if the ambient light fulfills one or more conditions, as will be outlined in further details below. The ambient light check specifically may comprise evaluating a suitability of the ambient light with regard to the analytical measurement. For example, the ambient light check may evaluate if the ambient light is suitable for performing the analytical measurement. In particular, the ambient light check may ensure that an intensity of the ambient light may be appropriate for performing the analytical measurement. Specifically, the ambient light check may ensure that the intensity of the ambient light is below and/or not exceeding a predetermined threshold value. Thus, typically, intense ambient light may lead to unwanted reflections or other disturbances which prevent the analytical measurement and/or which may reduce the accuracy of remission measurements used for determining the concentration of the at least one analyte in a bodily fluid. As an example, intense ambient light may be reflected by the optical test strip, a color reference card or both. By using the ambient light check, these uncertainties may be avoided or at least reduced. The ambient light check may also be advantageous in case the mobile device comprises a light source or illumination source for illuminating the optical test strip and/or the reagent test region. Thus, as an example, the mobile device may comprise at least one light emitting diode (LED) which may be used for illuminating the optical test strip and/or the reagent test region. The light source may define the illumination at the place of in the region which undergoes a detection reaction, such as a color change reaction, in the presence of the analyte to be detected. In this case, the ambient light check specifically may ensure that, via the illumination by the light source, the color values are not changed in such a way that these color values resulting from a combination of the ambient light and the illumination by the light source are outside the acceptance range or Gamut of the camera of the mobile device.

As an example, when the intensity of the ambient light exceeds the at least one threshold value, the analytical measurement may determine biased analyte concentration values. Thus, the ambient light check may ensure a correct determination of the concentration of the analyte in the bodily fluid. The ambient light check may specifically be performed by the mobile device automatically, i.e. without user interaction or triggering by the user, such as when starting a software app controlling the method.

The ambient light check, as outlined above, makes use of the at least one ambient light detector. Thus, the ambient light detector and the camera may be on opposing sides of the mobile device, such as of the smart phone. Thereby, as an example, the ambient light detector may directly detect ambient light from an artificial light source such as a ceiling lamp and/or from a natural light source such as the sun. Contrarily, a detector on the same side as the camera may not be capable of detecting the ambient light, since the mobile device itself may block the ambient light. Further, additionally or alternatively, the ambient light check may be performed without using a light source or an illumination source of the mobile device. Thus, the mobile device may comprise at least one light source, and for capturing the at least one image of at least a part of at least one reagent test region in step ii., the light source of the mobile device may be used, such as at least one light emitting diode (LED), e.g. for illuminating the reagent test region and measuring reflected and/or remitted light. For the ambient light check in step i., however, the light source of the mobile device may be kept switched off and/or may not be used. This implies the advantage of avoiding confusing the user which might be the case when switching on the light source repeatedly, e.g. at least once for the ambient light check and at least once for capturing the image of the reagent test region.

As further outlined above, the ambient light check comprises in step i.1, using the ambient light detector for determining at least one item of ambient light information and in step i.2, determining if at least one validity criterion on the item of ambient light information is fulfilled. The term "item of ambient light information" as used herein is a broad term and is to be given its ordinary and customary meaning to a person of ordinary skill in the art and is not to be limited to a special or customized meaning. The term specifically may refer, without limitation, to an item of information qualifying and/or quantifying the ambient light, such as an item of information indicating an intensity of the ambient light. The item of ambient light information may specifically indicate the intensity of the ambient light qualitatively and/or quantitatively. The item of ambient light information may be or may comprise at least one radiometric quantity and/or at least one photometric quantity. Alternatively and/or additionally, the item of ambient light information may comprise at least one dimensionless quantity, such as a bit information. The item of ambient light information may be determined by using at least one of the ambient light sensor and/or the additional camera. The radiometric quantity and/or the photometric quantity may be determined by using the additional camera of the mobile device. The dimensionless quantity, such as the bit information, may be determined by using the ambient light sensor.

The term "validity criterion" as used herein is a broad term and is to be given its ordinary and customary meaning to a person of ordinary skill in the art and is not to be limited to a special or customized meaning. The term specifically may refer, without limitation, to an arbitrary item of information qualifying and/or quantifying the at least one item of ambient light information. Thus, as an example, if the item of ambient light information has at least one predetermined value or is within at least one predetermined value range, the validity criterion may be fulfilled, whereas, if the item of ambient light information does not have the at least one predetermined value or does not lie within the at least one predetermined value range, the validity criterion may not be fulfilled. The validity criterion may indicate the validity of the item of ambient light information, such as by using one or more numerical values and/or by using one or more Boolean values and/or one or more digital values, such as "fulfilled" and "not fulfilled" or the like. As an example and as will be outlined in further detail below, in case the item of ambient light information is above or below a predetermined threshold value, the validity criterion may be set to a specific value. For example, one or more threshold values may be used, wherein, in case the item of ambient light information is above the threshold value, the validity criterion may be set to "not fulfilled", otherwise "fulfilled" or vice versa.

As outlined above, the method comprises in step ii., if the validity criterion is fulfilled, determining the concentration of the analyte in the bodily fluid by evaluating at least one image of at least a part of at least one reagent test region of an optical test strip having a sample of the bodily fluid applied thereto, the image being captured by the camera of the mobile device.

The term "optical test strip" as used herein is a broad term and is to be given its ordinary and customary meaning to a person of ordinary skill in the art and is not to be limited to a special or customized meaning. The term specifically may refer, without limitation, to an arbitrary element or device configured for performing a color formation reaction. The optical test strip may also be referred to as test strip or test element, wherein all three terms may refer to the same element. The optical test strip may particularly have a reagent test region containing at least one test chemical for detecting at least one analyte. The optical test strip, as an example, may comprise at least one substrate, such as at least one carrier, with the at least one reagent test region applied thereto or integrated therein. **In** particular, the optical test strip may further comprise at least one white area, such as a white field, specifically in a proximity to the reagent test region, for example enclosing or surrounding the reagent test region. The white area may be a separate field independently arranged on the substrate or carrier. However, additionally or alternatively, the substrate or carrier itself may be or may comprise the white area. As an example, the at least one carrier may be strip-shaped, thereby rendering the test element a test strip. These test strips are generally widely in use and available. One optical test strip may carry a single reagent test region or a plurality of reagent test regions having identical or different test chemicals comprised therein.

The term "reagent test region" as used herein is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the reagent test region having the test chemical comprised therein.

The term "capturing an image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "life stream" of images with the camera, wherein one or more of the images, automatically or by user interaction such as pushing a button, are stored and used as the at least one first image or the at least one second image, respectively. The image acquisition may be supported by a processor of the mobile device, and the storing of the images may take place in a data storage device of the mobile device. Alternatively or additionally, the captured images may fully or partially be transferred to a cloud system and the storing of the images may fully or partially take place in a data storage device of the cloud system.

The at least one image of the at least one part of the optical test strip specifically may comprise an image of at least a part of the reagent test region. Further, the image may comprise an image of other parts of the optical test strip, such as a white reference part of the optical test strip.

The capturing of the at least one image may comprise capturing at least one image with having the sample of the bodily fluid applied to the test strip and, further and optionally, such as before capturing the image with the sample applied to the test strip, capturing at least one image without having the sample of the body fluid applied to the test strip. The latter image specifically may be used for comparative purposes and may also be referred to as a "blank image" or "dry image". The sample application generally may take place, as an example, directly or indirectly, e.g. via at least one capillary element. The at least one image captured after sample application may typically also be referred to as the "wet image", even though the sample may have dried when the image is actually captured. The wet image typically may be taken after having waited for at least a predetermined waiting time, such as after five seconds or more, in order to allow for the color formation reaction to take place. Thus, as an example, the method may comprise, between taking the at least one optional dry image and the at least one wet image, waiting for at least a predetermined minimum amount of time. This predetermined minimum amount of time specifically may be sufficient for the color formation reaction to take place in the optical test strip. As an example, the minimum amount of waiting time may be at least 5 s.

The term "sample of the bodily fluid", also referred to as "sample", as used herein is a broad term and is to be given its ordinary and customary meaning to a person of ordinary skill in the art and is not to be limited to a special or customized meaning. The term specifically may refer, without limitation, to an arbitrary aliquot part or aliquant part of the bodily fluid. Specifically, the sample of the bodily fluid may refer to the part of the bodily fluid being applied to the optical test strip, more specifically to the reagent test region of the optical test strip.

As outlined above, the method, specifically step i.2, may further comprise comparing the at least one item of ambient light information with at least one threshold value. The term "threshold value" as used herein is a broad term and is to be given its ordinary and customary meaning to a person of ordinary skill in the art and is not to be limited to a special or customized meaning. The term specifically may refer, without limitation, to an arbitrary numerical value which may be used for the ambient light check. Specifically, the item of ambient light information may be compared with the at least one threshold value. The validity criterion may be determined based on the comparison of the item of ambient light information with the threshold value. The validity criterion may be fulfilled if one of the following conditions is fulfilled: the at least one item of ambient light information has a value below the threshold value; at least one item of ambient light information has a value not exceeding the threshold value. Alternatively or additionally, at least one first threshold value may be used for the ambient light check, wherein the validity criterion may be fulfilled if the at least one item of ambient light information has a value below or equal the first threshold value. Further, the ambient light check may use at least one second threshold value, wherein the validity criterion may be not fulfilled if the at least one item of ambient light information has a value above or equal to the second threshold value. **In** case the item of ambient light information may fall within an intermediate range being in between the first threshold value and the second threshold value, the validity criterion may set to "potentially unsecure". **In** this case, the user may be allowed to proceed with the analytical measurement and the analyte concentration may be labelled with a warning notice of being potentially inaccurate.

The threshold value may be a predetermined threshold value, such as a threshold value being equal for all mobile devices and/or a mobile device-specific threshold value. It may be possible to adapt the threshold value based on the analyte concentration determined by the method according to the present invention, such as by using one or more machine learning algorithms. As an example, for high analyte concentration values, which specifically may yield a dark color value, the threshold value may be adapted to allow a higher intensity of the ambient light. The threshold value may also be determined empirically or semi-empirically, such as by determining ambient light conditions under which the determining of the concentration of at least one analyte in a bodily fluid is still possible and by determining ambient light conditions under which the determining of the concentration of at least one analyte in a bodily fluid is disturbed or impeded by ambient light, such that, e.g., an accuracy of the measurement is below a predetermined accuracy threshold.

As outlined above, the at least one item of ambient light information may comprise at least one radiometric quantity and/or at least one photometric quantity. Specifically, the at least one item of ambient light information may comprise at least one of: an irradiance measurement value; a relative irradiance measurement value; a brightness measurement value; a relative brightness measurement value; an exposure measurement value; a relative exposure measurement value.

The method further comprises:
iii. if the validity criterion is not fulfilled, automatically preventing the determining of the concentration of the analyte in the bodily fluid.

Specifically, step iii. may comprise preventing, specifically automatically preventing, at least one of: capturing the image of the reagent test region; requesting the user to position the optical test strip within the field of view of the camera; requesting the user to apply the sample of the bodily fluid to the reagent test region of the optical test strip; pricking of a user's finger to apply the sample of the bodily fluid to the reagent test region of the optical test strip; evaluating the color formation reaction of the reagent test region.

Step ii. of the method may further comprise:
ii.1 capturing the at least one image of the at least one part of the at least one reagent test region having the sample of the bodily fluid applied thereto;
ii.2 determining at least one item of optical information indicative of the color formation reaction of the reagent test region; and
ii.3 deriving the concentration of the analyte by using the item of optical information.

The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction. The color formation may be detected by the mobile device, such as by a processor of the mobile device, and may be evaluated quantitatively, such as by deriving, from the at least one image, at least one parameter quantifying or characterizing the color formation of the reagent test region due to the presence of the analyte in the bodily fluid. As an example, one or more of the above-mentioned color values, such as at least one color value for one or more of the three colors R, G, B, may be used. Thus, the mobile device and specifically the processor of the mobile device may be configured for determining a color change by determining a change of one or more color values taking place due to the color formation reaction.

The term "item of optical information" as used herein is a broad term and is to be given its ordinary and customary meaning to a person of ordinary skill in the art and is not to be limited to a special or customized meaning. The term specifically may refer, without limitation, to an item of information comprising the at least one parameter quantifying or characterizing the color formation of the reagent test region due to the presence of the analyte in the bodily fluid. As an example, the item of optical information may be or may comprise a value of at least one color values derived from the image, such as an R, G or B value.

The at least one analyte concentration value may be determined from the color formation reaction of the reagent test region, specifically by using the item of optical information. For this purpose, the at least one image may be used. The analyte concentration value, as an example, may be a numerical value indicator of a result of the analytical measurement, such as indicative of the concentration of the at least one analyte in the sample, such as a blood glucose concentration.

The method step ii. may further comprise:
ii.4 prompting the user to apply the sample of the bodily fluid to the reagent test region.

The term "prompting" as used herein is a broad term and is to be given its ordinary and customary meaning to a person of ordinary skill in the art and is not to be limited to a special or customized meaning. The term specifically may refer, without limitation, to providing instructions by one or more of visual, audible and/or haptic means, requesting the user to perform one or more specific actions. The instructions may specifically be provided by the mobile device, such as via a display of the mobile device, via a loudspeaker of the mobile device, via a haptic feedback of the mobile device or the like. For example, verbal instructions and/or pictographic instructions may be prompted to the user on the display of the mobile device. Additionally or alternatively, the prompting may comprise playing a sound and/or playing spoken instructions via the loudspeakers of the mobile device.

Step ii. may further comprise:
ii.5 prompting the user, before performing step ii.1, to position the optical test strip within the field of view of the camera.

The method step ii. may further comprise:
ii.6 prompting the user, before performing step ii.1, to position at least one color reference card within the field of view of the camera, the color reference card comprising a plurality of color reference fields having known reference color values.

Steps ii.5 and ii.6 may be performed as separate steps or may fully or partially be combined, such as in one single step, such as in an alternative step ii.5 comprising prompting the user, before performing step ii.1, to position the optical test strip and the color reference card within the field of view of the camera.

The term "color reference card" as used herein is a broad term and is to be given its ordinary and customary meaning to a person of ordinary skill in the art and is not to be limited to a special or customized meaning. The term specifically may refer, without limitation, to an arbitrary item having, disposed therein or disposed thereon, such as on at least one surface, the plurality of color reference fields having known color properties or optical properties, such as having a plurality of colored fields having known reference color values. Further, the color reference card may comprise a plurality of gray reference fields having known gray levels. As an example, the color reference card may be a flat card comprising at least one substrate having, on at least one surface and/or disposed therein, the plurality of color reference fields having known color values and the plurality of gray reference fields having known gray levels. The substrate, specifically, may have a flat surface with the color reference fields and the gray reference fields disposed thereon. The substrate, as an example, may be or may comprise one or more of a paper substrate, a cardboard substrate, a plastic substrate, a ceramic substrate or a metal substrate. Laminate substrates are also possible. The substrate, as an example, may be sheet-like or flexible. It shall be noted, however, that the substrate may also be implemented into an article of use, such as into a wall of a box, a vial, a container, a medical consumable, such as a test strip, or the like. The color reference card may also fully or partially be integrated into the optical test strip. The at least one image of at least the part of the reagent test region of the optical test strip may fully or partially comprise an image of at least one part of the color reference card.

Specifically, step ii.3 may be performed by using at least one measured reference color value derived from the image. For example, by using at least one measured reference color value of one or more color reference fields, a color correction may be derived. The color correction may transform measured reference color values into known reference color values. Thus, the color correction may be configured for correcting measured color values in the image. The color correction may also be applied to the color value of the reagent test region, specifically to the item of optical information of the color formation reaction of the reagent test region.

The camera and the ambient light detector may be located on opposing sides of the mobile device. The ambient light sensor may be comprised by at least one display of the mobile device. The ambient light sensor may comprise at least one photosensitive element, specifically at least one of a photodiode, a phototransistor, a photoconductor, a semiconductor element.

As outlined above, the additional camera may comprise at least one front camera being located on the front side of the mobile device, the front side of the mobile device comprising at least one operation element of the mobile device, specifically at least one touchscreen. The operation element of the mobile device, specifically the touchscreen, may also form part of the display of the mobile device.

The method may specifically be computer-implemented. The computer-implementation may comprise a software application running on the mobile device, wherein the software application may comprise, after starting the software application, guiding the user through a measurement sequence, wherein the measurement sequence may comprise at least step ii., wherein step i. may be performed fully or partially before and/or during the measurement sequence. Specifically, the software application may be configured for prompting the user, such as for providing instructions via the mobile device.

In a further aspect of the present invention, a mobile device having at least one camera is disclosed, the mobile device specifically further comprising at least one processor, the mobile device further comprising at least one ambient light detector, the ambient light detector comprising at least one of an ambient light sensor and an additional camera, wherein the mobile device is configured for performing the method according to the present invention, such as according to any one of the embodiments disclosed above and/or according to any one of the embodiments disclosed in further detail below.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The processor specifically may be configured, such as by software programming, for performing and/or supporting the method steps of the method. Specifically, the processor may be configured for supporting the capturing of the at least one image of the at least one part of the at least one reagent test region of the optical test strip by using the camera. The processor may further be configured for determining at least one analyte concentration value from color formation of the reagent test region, such as by evaluating the image, deriving the at least one item of optical information from the image and by transforming the at least one item of optical information into the at least one analyte concentration value. The processor specifically may further be configured for supporting one or more or all of steps i., and ii., and optionally step iii., of the method, such as for performing the ambient light check, such as for determining the item of ambient light information, such as for determining the validity criterion on the ambient light information, such as for evaluating the image of the part of the reagent test region of the optical test strip and for preventing the determining of the concentration of the analyte if the validity criterion may not be fulfilled. The processor may further be configured for supporting sample application to the optical test strip, such as by providing user guidance, e.g. in a visual format and stuff or in an audible format. The processor may further be configured for supporting the capturing of the at least one image, e.g. by automatically detecting the optical test strip or a part thereof in a field of view and/or by prompting the user to capture the image.

**In** a further aspect of the present invention a kit is disclosed, comprising the mobile device according to the present invention, such as according to any one of the embodiments disclosed above and/or according to any one of the embodiments disclosed in further detail below, the kit further comprising least one optical test strip having at least one reagent test region.

The kit may further comprise at least one color reference card, the color reference card comprising a plurality of color reference fields having known reference color values.

**In** a further aspect of the invention, a computer program is disclosed comprising instructions which, when the program is executed by the mobile device according to the present invention, such as according to any one of the embodiments disclosed above and/or according to any one of the embodiments disclosed in further detail below, cause the mobile device, specifically a processor of the mobile device, to perform the method according to the present invention, such as according to any one of the embodiments disclosed above and/or according to any one of the embodiments disclosed in further detail below.

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

In a further aspect of the present invention, a computer-readable storage medium is disclosed comprising instructions which, when executed by the mobile device according to the present invention, such as according to any one of the embodiments disclosed above and/or according to any one of the embodiments disclosed in further detail below, cause the mobile device, specifically a processor of the mobile device, to perform the method according to the present invention, such as according to any one of the embodiments disclosed above and/or according to any one of the embodiments disclosed in further detail below.

As used herein, the term "computer-readable storage medium" specifically may refer to a non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The method and devices according to the present invention provide a large number of advantages over similar methods and devices known in the art. Specifically, compared to methods and devices known in the art, the method and devices as described herein may increase flexibility of analytical measurements. In particular, by performing the ambient light check using the ambient light detector, it may be possible to perform the method of determining the concentration of the at least one analyte in the bodily fluid on a vast number of different mobile devices. Specifically by using alternatively or additionally one or more of the ambient light sensor and the additional camera, the method may be performed on a large variety of different mobile devices.

Further, the method and devices may improve the user-friendliness of analytical measurement. The method comprises preventing the determining of the concentration of the analyte in the bodily fluid if the validity criterion may not be fulfilled. Therefore, the method may provide means for preventing the user to unnecessarily apply the sample of the bodily fluid onto the reagent test region of the optical test strip. Further, the method according to the present invention may also save resources in case the optical test strip may not be used when the user is prevented from applying the sample of the bodily fluid onto the optical test strip.

Moreover, the method and devices according to the present invention may improve accuracy and safety of analytical measurements. By using the ambient light check together with the color reference card, the determining of the concentration of the at least one analyte in the bodily fluid may become more reliable and accurate. False and/or biased results of the analytical measurement may become more unlikely since appropriate lighting conditions may be ensured by the ambient light check. Further, the method may allow using the camera of the mobile device in an automatic mode for the determining the concentration of the analyte of the bodily fluid whereas the ambient light check may use the ambient light detector, such as one or more of the ambient light sensor and the additional camera. Thus, the ambient light check may be performed before and/or during the measurement sequence which may use the camera of the mobile device in the automatic mode.

The methods and devices according to the present invention may enable performing the ambient light check when determining the concentration of the at least one analyte in the bodily fluid in cases, where the color reference card is required to be used together with the optical test strip. The method may enable applying the ambient light check while using the automatic mode of the camera to capture the at least one image of the part of the reagent test region of the optical test strip. Specifically, the method may allow performing the ambient light check while using the full automatic mode of the camera of the mobile device to capture the image of the optical test strip.

The method and devices according to the present invention may provide an alternative means for the ambient light check by using the ambient light sensor and/or the additional camera instead of using the camera of the mobile device. The ambient light sensor and/or the additional camera may be used in parallel to the camera of the mobile device, and, thus, may allow performing the ambient light check before and/or during the measurement sequence.

The ambient light check may specifically be performed in parallel to the capturing of the at least one image of the part of the reagent test region of the optical test strip. Thus, prompting the user, specifically providing instructions to the user, may be performed simultaneously to ensuring the ability to perform the analytical measurement. Further, the ambient light check may be provided for analytical measurements requiring using a color reference card together with the optical test strip, since these methods generally require capturing the image in the automatic mode of the camera. Alternatively or additionally, the ambient light check may also be used for analytical measurements which are performed on the mobile device at least partially using the manual mode of the camera.

In case the optical test strip without the color reference card may be used, the ambient light sensor and/or the additional camera may be used additionally to the camera of the mobile device for the ambient light check. By doing so, data on the ambient light may be collected and analyzed via at least one cloud computing system to further improve the ambient light check. In case the color reference card may be used together with the optical test strip, the ambient light check may be directly implemented since no alternative method is known in the art.

As it is apparent from any one of embodiments described above and/or any one of the embodiments disclosed in further detail below, the method and devices according to the present invention may provide an ambient light check for the case the color reference card may be used together with the optical test strip and the image of the reagent test region of the optical test strip may be captured in an automatic mode of the camera. The method may comprise some uncertainties regarding the ambient light check, but so far, may be regarded the best alternative for checking the ambient light situation using the automatic mode of the camera.

As outlined above, the method may fully or partially be computer-implemented and/or computer-controlled. Specifically, steps i. and ii. may be computer-implemented and may be performed e.g. by software running on the processor of the mobile device, such as a software app. Further, one or more or all of the additional optional method steps may be computer-implemented, e.g. by the software running on the processor, such as step iii. Further, the capturing of the at least one image in step ii.1 may be computer-controlled by the software running on the processor, and steps ii.2 to ii.3 and optionally also any one or even all of steps ii.4 to ii.5 may be computer-implemented by the same software. The software running on the processor may also be referred to as a software application or software app and may comprise one or more software components. The software, as outlined above, may also guide the user through the measurement sequence.

The software or computer program generally may include computer-executable instructions for performing the method according to the present invention in one or more of the embodiments enclosed herein when the program is executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a kit and a mobile device configured for performing a method of determining the concentration of at least one analyte in a bodily fluid in a perspective view;
- Figure 2: shows a flow chart of an embodiment of a method of determining the concentration of at least one analyte in a bodily fluid by using a mobile device; and
- Figure 3: shows a flow chart of an embodiment of an ambient light check.

### Detailed description of the embodiments

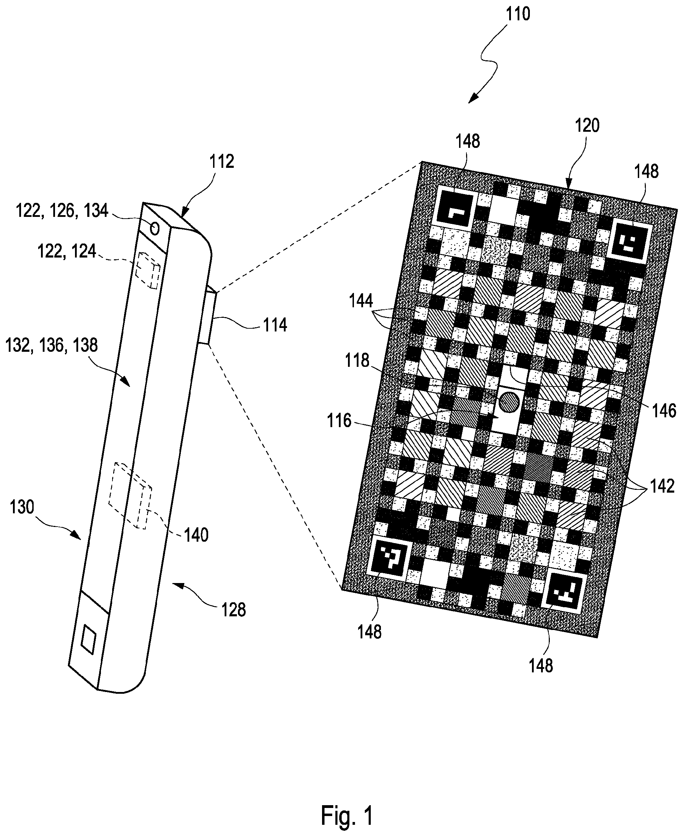

In Figure 1, an exemplary embodiment of a kit 110 is shown in a perspective view. The kit 110 comprises a mobile device 112 having at least one camera 114. The kit 110 further comprises at least one optical test strip 116 having at least one reagent test region 118. The kit 110 may comprise at least one color reference card 120.

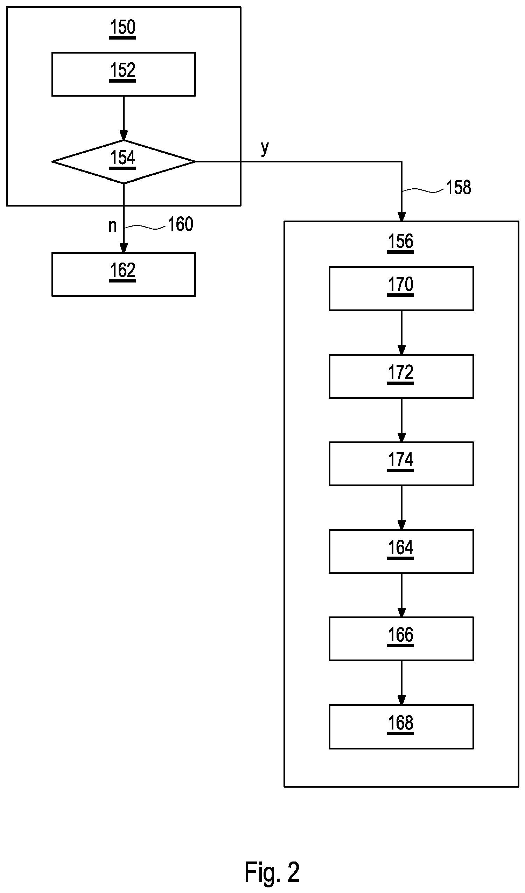

The mobile device 112 further comprises at least one ambient light detector 122. The ambient light detector 122 comprises at least one of an ambient light sensor 124 and an additional camera 126. The mobile device 112 is configured for performing a method of determining the concentration of at least one analyte in a bodily fluid. The method will be described with reference to exemplary embodiments shown in a flowchart illustrated in Figure 2. Thus, reference may be made to the description of Figure 2. As can be seen in Figure 1, the camera 114 and the ambient light detector 122 may be located on opposing sides of the mobile device 112. For example, the camera 114 may be located on a back side 128 of the mobile device 112. The back side may 128 refer to the side of the mobile device 112 facing one or more of the optical test strip 116, the reagent test region 118 and the color reference card 120. The ambient light detector 122 may be located on a front side 130 of the mobile device 112. The front side 130 of the mobile device 112 may face a user of the mobile device 112.

Further, the mobile device 112 may comprise at least one display 132. The ambient light sensor 124 may be comprised by the display 132 of the mobile device 112. The ambient light sensor 124 may comprise at least one photosensitive element, specifically at least one of a photodiode; a phototransistor; a photoconductor; a semiconductor element. The display 132 and, thus, the ambient light sensor 124, may be located on the front side 130 of the mobile device 112.

The additional camera 126 may comprise at least one front camera 134. The front camera 134 may be located on the front side 130 of the mobile device 112. The front side 130 of the mobile device 112 may comprise at least one operation element 136 of the mobile device 112. Specifically, the operation element 136 may be at least one touchscreen 138. The operation element 136, specifically the touchscreen 138, may form part of the display 132 of the mobile device 112.

The mobile device 112 may further comprise at least one processor 140. The processor 140 may be configured, such as by software programming, for performing and/or supporting one or more or even all of the method steps of the method of determining the concentration of at least one analyte in a bodily fluid. Specifically, the processor 140 may be configured for supporting capturing of at least one image of at least one part of the at least one reagent test region 118 of the optical test 116 strip by using the camera 114. The processor 140 may further be configured for determining at least one analyte concentration value from color formation of the reagent test region 118. The processor 140 may further be configured for performing an ambient light check 178 and for preventing the determining of the concentration of the analyte if a validity criterion may not be fulfilled. The processor 140 may be configured for supporting sample application to the optical test strip 116, such as by providing user guidance, e.g. in a visual format and stuff or in an audible format. For example, the user guidance may be provided via the display 132 of the mobile device 112 in a visual format.

The color reference card 120 may comprise a plurality of color reference fields 142 having known reference color values. The color reference card 120 may further comprise a plurality of gray reference fields 144 having known gray levels. The plurality of color reference fields 142 and the plurality of gray reference fields 144 may be disposed on a surface of the color reference card 120, specifically such that the plurality of color reference fields 142 and the plurality of gray reference fields 144 may be visible when capturing the image of the optical test strip 116.

The color reference card 120 may fully or partially be integrated into the optical test strip 116. Alternatively or additionally, the color reference card 120 may comprise at least one window element 146, wherein the window element 146 may be configured for receiving the optical test strip 116. The optical test strip 116 may be visible to the camera 114 of the mobile device 112 when the optical test strip 116 is received by the window element 146 of the color reference card 120.

The color reference card 120 may further comprise at least one position marker 148. The mobile device 112, specifically the processor 140 of the mobile device 112, may be configured for identifying a position and/or an orientation of the color reference card 120 by recognizing the position marker 148 in the at least one image captured by the camera 114.

Figure 2 shows a flow chart of an exemplary embodiment of a method of determining the concentration of at least one analyte in a bodily fluid by using the mobile device 112, the mobile device 112 having the at least one camera 114 and the at least one ambient light detector 122, the ambient light detector 122 comprising at least one of an ambient light sensor 124 and an additional camera 126.

The method comprises the following steps. It may further be possible to perform one, more than one or even all of the method steps once or repeatedly. The method may comprise additional method steps that are not listed.

The method comprises:
i. (denoted by reference number 150) performing the at least one ambient light check 178, the ambient light check 178 comprising:
   i.1 (denoted by reference number 152) using the ambient light detector 122 for determining at least one item of ambient light information; and
   i.2 (denoted by reference number 154) determining if at least one validity criterion on the item of ambient light information is fulfilled;
ii. (denoted by reference number 156) if the validity criterion is fulfilled, determining the concentration of the analyte in the bodily fluid by evaluating at least one image of at least a part of the at least one reagent test region 118 of the optical test strip 116 having a sample of the bodily fluid applied thereto, the image being captured by the camera 114 of the mobile device 112.

The item of ambient light information determined in step i.2 may specifically comprise one or more of: an irradiance measurement value; a relative irradiance measurement value; a brightness measurement value; a relative brightness measurement value; an exposure measurement value; a relative exposure measurement value.

Specifically, step i.2 may comprise comparing the at least one item of ambient light information with at least one threshold value. Based on the comparison of the item of ambient light information with the at least one threshold value, the at least one validity criterion may be determined. The validity criterion may be fulfilled if one of the following conditions is fulfilled: the at least one item of ambient light information has a value below the threshold value; at least one item of ambient light information has a value not exceeding the threshold value.

As illustrated in Figure 2, step i.2 (denoted by reference number 154) may be a branching point of the method. The branching point may indicate a condition query, such as deciding between a first branch 158 and a second branch 160. The condition query in step i.2 may use the validity criterion. The validity criterion may comprise a numerical value indicating the validity of the item of ambient light information. For example, the validity criterion may comprise a Boolean value such as "fulfilled" ("y") and "not fulfilled" ("n") or the like. As an example, in case the item of ambient light information may be below and/or may not exceed the threshold value, the validity criterion may be set to "fulfilled" and the first branch 158 may lead to step ii. of the method. The second branch 160 may indicate a non-valid item of ambient light information, such as an item of ambient light information being above the threshold value, and the validity criterion may be set to "not fulfilled". Thus, the second branch 160 may lead to optional step iii. of the method.

The method further comprises:
iii. (denoted by reference number 162) if the validity criterion is not fulfilled, automatically preventing the determining of the concentration of the analyte in the bodily fluid.

Specifically, step iii. may comprise preventing at least one of: capturing the image of the reagent test region 118; requesting the user to position the optical test strip 116 within a field of view of the camera 114; requesting the user to apply the sample of the bodily fluid to the reagent test region 118 of the optical test strip 116; pricking of a user's finger to apply the sample of the bodily fluid to the reagent test region 118 of the optical test strip 116; evaluating a color formation reaction of the reagent test region 118.

The method step ii. may further comprise:
ii.1 (denoted by reference number 164) capturing the at least one image of the at least one part of the at least one reagent test region 118 having the sample of the bodily fluid applied thereto;
ii.2 (denoted by reference number 166) determining at least one item of optical information indicative of a color formation reaction of the reagent test region 118; and
ii.3 (denoted by reference number 168) deriving the concentration of the analyte by using the item of optical information.

Step ii.3 may specifically be performed by using at least one measured reference color value derived from the image. For example, by using at least one measured reference color value of one or more color reference fields 142, a color correction may be derived. The color correction may transform measured reference color values into known reference color values. Thus, the color correction may be configured for correcting measured color values in the image. The color correction may also be applied to the color value of the reagent test region 118, specifically to the item of optical information of the color formation reaction of the reagent test region 118.

Step ii. may further comprise one or more of the following steps:
ii.4 (denoted by reference number 170) prompting the user to apply the sample of the bodily fluid to the reagent test region 118.
ii.5 (denoted by reference number 172) prompting the user, before performing step ii.1, to position the optical test strip 116 within the field of view of the camera 114.
ii.6 (denoted by reference number 174) prompting the user, before performing step ii.1, to position the at least one color reference card 120 within the field of view of the camera 114, the color reference card 120 comprising the plurality of color reference fields 142 having known reference color values.

The prompting may specifically comprise providing instructions by the mobile device 112, such as via the display 132 of the mobile device 112, via a loudspeaker of the mobile device 112, via a haptic feedback of the mobile device 112 or the like. For example, verbal instructions and/or pictographic instructions may be prompted to the user on the display 132 of the mobile device 112. Additionally or alternatively, the prompting may comprise playing a sound and/or playing spoken instructions via the loudspeakers of the mobile device 112.

Further, the method may be computer-implemented. The computer-implementation may comprise a software application running on the mobile device 112. The software application may comprise, after starting the software application, guiding the user through a measurement sequence 176, wherein the measurement sequence 176 may comprise at least step ii. The measurement sequence 176 may further comprise one or more of steps ii.1, ii.2, ii.3, ii.4, ii.5 and/or ii.6. Step i. may be performed fully and/or partially before and/or during the measurement sequence 176.

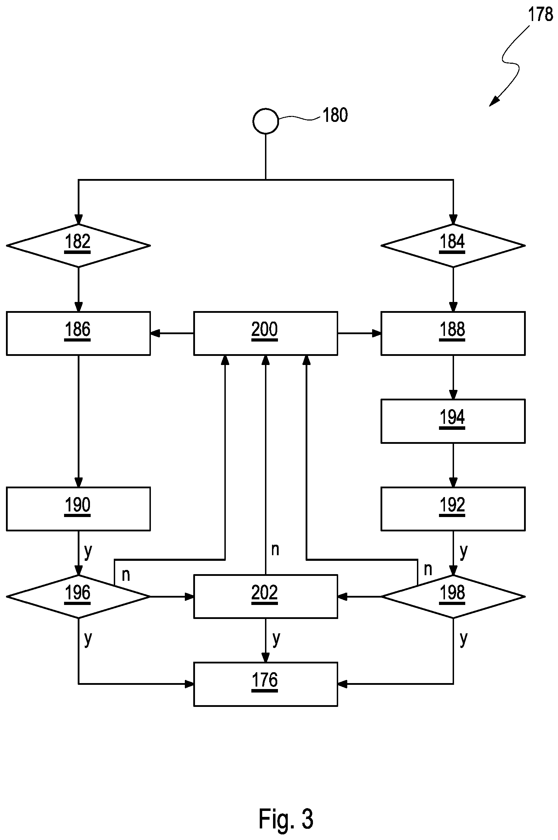

In Figure 3, a flowchart of an exemplary embodiment of the ambient light check 178 is shown. The ambient light check 178 may be started by a user request 180, such as a user request 180 for the measurement sequence 176, for example a measurement sequence 176 comprising determining the concentration of the analyte in the bodily fluid. The ambient light check 178 may specifically be performed before the measurement sequence 176 starts. Additionally and/or alternatively, the ambient light 178 check may also be performed during the measurement sequence 176.

Followed by the user request 180, the ambient light check 178 may comprise checking if information from the ambient light sensor 124 (denoted by reference number 182) and from the additional camera 126 (denoted by reference number 184) is available. If at least one of these information is available, the ambient light check 178 may comprise reading the information from the ambient light sensor 124 (denoted by reference number 186) and from the additional camera 126 (denoted by reference number 188). As an example, the information may comprise sensor information for the ambient light sensor 124 and pixel information for the additional camera 126, respectively.

In further steps, the ambient light check 178 may comprise determining the item of ambient light information by using the ambient light sensor 124 (denoted by reference number 190) and by using the additional camera 126 (denoted by reference number 192). As for the additional camera 126, the item of ambient light information may take into account meta information from the image capturing (denoted by reference number 194), such as an ISO-value, an aperture of the additional camera 126 and/or a exposure time. For example, the pixel information from the additional camera 126 may comprise an exposure value, wherein the exposure value may be used for deriving a relative luminance using the meta information from the additional camera 126.

The ambient light 176 may further comprise comparing the item of ambient light information with the at least one threshold value for each of the item of ambient light information determined by using ambient light sensor 124 (denoted by reference number 196) and the additional camera 126 (denoted by reference number 198). The threshold value may be a predetermined threshold value, such as a threshold value being predetermined by laboratory measurements. Based on the comparison of the item of ambient light information with the threshold value, the at least one validity criterion may be determined.

In case the validity criterion may indicate an invalid item of ambient light information ("N"), such as an item of ambient light information exceeding the threshold value, the ambient light check 178 may prevent the measurement sequence 176 and may prompt the user to change the ambient light situation (denoted by reference number 200).

Additionally and/or alternatively, the ambient light check 178 may further comprise combining the independent ambient light assessments from the ambient light sensor 124 and the additional camera 126 for determining the at least one validity criterion (denoted by reference number 202). For example, if both assessments of the validity criterion on the item of ambient light information match ("Y"), the ambient light check 178 may allow performing the measurement sequence 176. If the assessments of the validity criterion on the item of ambient light information do not match ("N"), the ambient light check 178 may prevent performing the measurement sequence 176 and may prompt the user to change the ambient light situation (denoted by reference number 200). The combination of ambient light assessments may be possible if both, the ambient light sensor 124 and the additional camera 126 may be able to determine the item of ambient light situation.

The ambient light check 178 may use only one of the validity criteria determined by using one of the ambient light sensor 124 and the additional camera 126 if only one of the ambient light sensor 124 and the additional camera 126 is available. In case the validity criterion may indicate a valid item of ambient light information ("Y"), such as an item of ambient light information being below or not exceeding the threshold value, the ambient light check 178 may allow the measurement sequence 176.

### List of reference numbers

- 110: kit
- 112: mobile device
- 114: camera
- 116: optical test strip
- 118: reagent test region
- 120: color reference card
- 122: ambient light detector
- 124: ambient light sensor
- 126: additional camera
- 128: back side
- 130: front side
- 132: display
- 134: front camera
- 136: operation element
- 138: touchscreen
- 140: processor
- 142: color reference field
- 144: gray reference field
- 146: window element
- 148: position marker
- 150: performing at least one ambient light check
- 152: using the ambient light detector for determining at least one item of ambient light information
- 154: determining if at least one validity criterion on the item of ambient light information is fulfilled
- 156: determining the concentration of the analyte in the bodily fluid
- 158: first branch
- 160: second branch
- 162: preventing the determining of the concentration of the analyte in the bodily fluid
- 164: capturing the at least one image
- 166: determining at least one item of optical information
- 168: deriving the concentration of the analyte
- 170: prompting a user to apply the sample of the bodily fluid
- 172: prompting a user to position the optical test strip
- 174: prompting a user to position the at least one color reference card
- 176: measurement sequence
- 178: ambient light check
- 180: user request
- 182: checking if information from the ambient light sensor is available
- 184: checking if information from the additional camera is available
- 186: reading the information from the ambient light sensor
- 188: reading the information from the additional camera
- 190: determining the item of ambient light information by using the ambient light sensor
- 192: determining the item of ambient light information by using the additional camera
- 194: taking into account meta information from the image capturing
- 196: comparing the item of ambient light information determined by using the ambient light sensor with the threshold value
- 198: comparing the item of ambient light information determined by using the additional camera with the threshold value
- 200: prompt the user to change ambient light situation
- 202: combining independent ambient light assessments

## Claims

1. A method of determining the concentration of at least one analyte in a bodily fluid by using a mobile device (112), the mobile device (112) having at least one camera (114) and at least one ambient light detector (122), the ambient light detector (122) comprising at least one of an ambient light sensor (124) and an additional camera (126), the method comprising:
i. performing at least one ambient light check (178), the ambient light check (178) comprising:
i.1 using the ambient light detector (122) for determining at least one item of ambient light information; and
i.2 determining if at least one validity criterion on the item of ambient light information is fulfilled;
ii. if the validity criterion is fulfilled, determining the concentration of the analyte in the bodily fluid by evaluating at least one image of at least a part of at least one reagent test region (118) of an optical test strip (116) having a sample of the bodily fluid applied thereto, the image being captured by the camera (114) of the mobile device (112).;
wherein the method further comprises:
iii. if the validity criterion is not fulfilled, automatically preventing the determining of the concentration of the analyte in the bodily fluid.

2. The method according to the preceding claim, wherein step i.2 comprises comparing the at least one item of ambient light information with at least one threshold value.

3. The method according to the preceding claim, wherein the validity criterion is fulfilled if one of the following conditions is fulfilled: the at least one item of ambient light information has a value below the threshold value; at least one item of ambient light information has a value not exceeding the threshold value.

4. The method according to any one of the preceding claims, wherein step iii. comprises preventing at least one of: capturing the image of the reagent test region (118); requesting a user to position the optical test strip (116) within a field of view of the camera (114); requesting a user to apply the sample of the bodily fluid to the reagent test region (118) of the optical test strip (116); pricking of a user's finger to apply the sample of the bodily fluid to the reagent test region (118) of the optical test strip (116); evaluating a color formation reaction of the reagent test region (118).

5. The method according to any one of the preceding claims, wherein step ii. comprises:
ii.1 capturing the at least one image of the at least one part of the at least one reagent test region (118) having the sample of the bodily fluid applied thereto;
ii.2 determining at least one item of optical information indicative of a color formation reaction of the reagent test region (118); and
ii.3 deriving the concentration of the analyte by using the item of optical information.

6. The method according to the preceding claim, step ii. further comprising at least one of:
ii.4 prompting a user to apply the sample of the bodily fluid to the reagent test region (118);
ii.5 prompting a user, before performing step ii.1, to position the optical test strip (116) within a field of view of the camera (114);
ii.6 prompting a user, before performing step ii.1, to position at least one color reference card (120) within a field of view of the camera (114), the color reference card (120) comprising a plurality of color reference fields (142) having known reference color values.

7. The method according to any one of the preceding claims, wherein the camera (114) and the ambient light detector (122) are located on opposing sides of the mobile device (112).

8. The method according to any one of the preceding claims, wherein the ambient light sensor (124) is comprised by at least one display (132) of the mobile device (112).

9. The method according to any one of the preceding claims, wherein the additional camera (126) comprises at least one front camera (134) being located on a front side (130) of the mobile device (112), the front side (130) of the mobile device (112) comprising at least one operation element (136).

10. The method according to anyone of the preceding claims, wherein the method is computer-implemented, wherein the computer-implementation comprises a software application running on the mobile device (112), wherein the software application comprises, after starting the software application, guiding a user through a measurement sequence (176), wherein the measurement sequence (176) comprises at least step ii., wherein step i. is performed fully or partially before and/or during the measurement sequence (176).

11. A mobile device (112) having at least one camera (114), the mobile device (112) further comprising at least one ambient light detector (122), the ambient light detector (122) comprising at least one of an ambient light sensor (124) and an additional camera (126), wherein the mobile device (112) is configured for performing the method according to any one of the preceding claims.

12. A kit (110), comprising the mobile device (112) according to the preceding claim, the kit (110) further comprising least one optical test strip (116) having at least one reagent test region (118).

13. A computer program comprising instructions which, when the program is executed by the mobile device (112) according to any one of the preceding claims referring to a mobile device (112), cause the mobile device (112) to perform the method according to any one of the preceding claims referring to a method.

14. A computer-readable storage medium comprising instructions which, when executed by the mobile device (112) according to any one of the preceding claims referring to a mobile device (112), cause the mobile device (112) to perform the method according to any one of the preceding claims referring to a method.

## Patentansprüche

1. Verfahren zum Bestimmen der Konzentration mindestens eines Analyten in einer Körperflüssigkeit unter Verwendung einer mobilen Vorrichtung (112), wobei die mobile Vorrichtung (112) mindestens eine Kamera (114) und mindestens einen Umgebungslichtdetektor (122) aufweist, wobei der Umgebungslichtdetektor (122) mindestens eines von einem Umgebungslichtsensor (124) und einer zusätzlichen Kamera (126) umfasst, wobei das Verfahren Folgendes umfasst:
i. Durchführen mindestens einer Umgebungslichtprüfung (178), wobei die Umgebungslichtprüfung (178) Folgendes umfasst:
i.1 Verwenden des Umgebungslichtdetektors (122) zum Bestimmen mindestens eines Elements von Umgebungslichtinformationen; und
i.2 Bestimmen, ob mindestens ein Gültigkeitskriterium zu dem Element von Umgebungslichtinformationen erfüllt wird;
ii. wenn das Gültigkeitskriterium erfüllt wird, Bestimmen der Konzentration des Analyten in der Körperflüssigkeit durch Auswerten mindestens eines Bildes von mindestens einem Teil mindestens einer Reagenztestregion (118) eines optischen Teststreifens (116), auf die eine Probe der Körperflüssigkeit aufgebracht ist, wobei das Bild mit der Kamera (114) der mobilen Vorrichtung (112) aufgenommen wird;
wobei das Verfahren ferner Folgendes umfasst:
iii. wenn das Gültigkeitskriterium nicht erfüllt wird, automatisches Verhindern des Bestimmens der Konzentration des Analyten in der Körperflüssigkeit.

2. Verfahren nach dem vorstehenden Anspruch, wobei Schritt i.2 das Vergleichen des mindestens einen Elements von Umgebungslichtinformationen mit mindestens einem Schwellenwert umfasst.

3. Verfahren nach dem vorstehenden Anspruch, wobei das Gültigkeitskriterium erfüllt wird, wenn eine der folgenden Bedingungen erfüllt wird: das mindestens eine Element von Umgebungslichtinformationen weist einen Wert unter dem Schwellenwert auf; mindestens ein Element von Umgebungslichtinformationen weist einen Wert auf, der den Schwellenwert nicht überschreitet.

4. Verfahren nach einem der vorstehenden Ansprüche, wobei Schritt iii. das Verhindern mindestens eines von Folgendem umfasst: Aufnehmen des Bildes von der Reagenztestregion (118); Auffordern eines Benutzers, den optischen Teststreifen (116) innerhalb eines Sichtfeldes der Kamera (114) zu positionieren; Auffordern eines Benutzers, die Probe der Körperflüssigkeit auf die Reagenztestregion (118) des optischen Teststreifens (116) aufzubringen; Stechen in den Finger eines Benutzers, um die Probe der Körperflüssigkeit auf die Reagenztestregion (118) des optischen Teststreifens (116) aufzubringen; Auswerten einer Farbbildungsreaktion der Reagenztestregion (118).

5. Verfahren nach einem der vorstehenden Ansprüche, wobei Schritt ii. Folgendes umfasst:
ii.1 Aufnehmen des mindestens einen Bildes von dem mindestens einen Teil der mindestens einen Reagenztestregion (118), auf den die Probe der Körperflüssigkeit aufgebracht ist;
ii.2 Bestimmen mindestens eines Elements von optischen Informationen, die eine Farbbildungsreaktion der Reagenztestregion (118) angeben; und
ii.3 Ableiten der Konzentration des Analyten unter Verwendung des Elements von optischen Informationen.

6. Verfahren nach dem vorstehenden Anspruch, wobei Schritt ii. ferner mindestens eines von Folgendem umfasst:
ii.4 Veranlassen eines Benutzers, die Probe der Körperflüssigkeit auf die Reagenztestregion (118) aufzubringen;
ii.5 Veranlassen eines Benutzers, vor dem Durchführen von Schritt ii.1 den optischen Teststreifen (116) innerhalb eines Sichtfeldes der Kamera (114) zu positionieren;
ii.6 Veranlassen eines Benutzers, vor dem Durchführen von Schritt ii.1 mindestens eine Farbreferenzkarte (120) innerhalb eines Sichtfeldes der Kamera (114) zu positionieren, wobei die Farbreferenzkarte (120) eine Vielzahl von Farbreferenzfeldern (142) mit bekannten Referenzfarbwerten umfasst.

7. Verfahren nach einem der vorstehenden Ansprüche, wobei sich die Kamera (114) und der Umgebungslichtdetektor (122) auf gegenüberliegenden Seiten der mobilen Vorrichtung (112) befinden.

8. Verfahren nach einem der vorstehenden Ansprüche, wobei der Umgebungslichtsensor (124) von mindestens einer Anzeige (132) der mobilen Vorrichtung (112) umfasst ist.

9. Verfahren nach einem der vorstehenden Ansprüche, wobei die zusätzliche Kamera (126) mindestens eine vordere Kamera (134) umfasst, die sich auf einer Vorderseite (130) der mobilen Vorrichtung (112) befindet, wobei die Vorderseite (130) der mobilen Vorrichtung (112) mindestens ein Betätigungselement (136) umfasst.

10. Verfahren nach einem der vorstehenden Ansprüche, wobei das Verfahren computerimplementiert ist, wobei die Computerimplementierung eine Softwareanwendung umfasst, die auf der mobilen Vorrichtung (112) läuft, wobei die Softwareanwendung nach dem Starten der Softwareanwendung das Leiten eines Benutzers durch eine Messabfolge (176) umfasst, wobei die Messabfolge (176) mindestens Schritt ii. umfasst, wobei Schritt i. vollständig oder teilweise vor und/oder während der Messabfolge (176) durchgeführt wird.

11. Mobile Vorrichtung (112) mit mindestens einer Kamera (114), wobei die mobile Vorrichtung (112) ferner mindestens einen Umgebungslichtdetektor (122) umfasst, wobei der Umgebungslichtdetektor (122) mindestens eines von einem Umgebungslichtsensor (124) und einer zusätzlichen Kamera (126) umfasst, wobei die mobile Vorrichtung (112) dafür ausgebildet ist, das Verfahren nach einem der vorstehenden Ansprüche auszuführen.

12. Kit (110), umfassend die mobile Vorrichtung (112) nach dem vorstehenden Anspruch, wobei der Kit (110) ferner mindestens einen optischen Teststreifen (116) mit mindestens einer Reagenztestregion (118) umfasst.

13. Computerprogramm, umfassend Anweisungen, die beim Ausführen des Programms von der mobilen Vorrichtung (112) nach einem der vorstehenden Ansprüche, die sich auf eine mobile Vorrichtung (112) beziehen, die mobile Vorrichtung (112) veranlassen, das Verfahren nach einem der vorstehenden Ansprüche, die sich auf ein Verfahren beziehen, auszuführen.

14. Computerlesbares Speichermedium, umfassend Anweisungen, die beim Ausführen von der mobilen Vorrichtung (112) nach einem der vorstehenden Ansprüche, die sich auf eine mobile Vorrichtung (112) beziehen, die mobile Vorrichtung (112) veranlassen, das Verfahren nach einem der vorstehenden Ansprüche, die sich auf ein Verfahren beziehen, auszuführen.

## Revendications

1. Procédé de détermination de la concentration d'au moins un analyte dans un fluide corporel en utilisant un dispositif mobile (112), le dispositif mobile (112) ayant au moins une caméra (114) et au moins un détecteur de lumière ambiante (122), le détecteur de lumière ambiante (122) comprenant au moins un parmi un capteur de lumière ambiante (124) et une caméra supplémentaire (126), le procédé comprenant :
i. la réalisation d'au moins une vérification de lumière ambiante (178), la vérification de lumière ambiante (178) comprenant :
i.1 l'utilisation du détecteur de lumière ambiante (122) pour déterminer au moins un élément d'informations de lumière ambiante ; et
i.2 la détermination si au moins un critère de validité sur l'élément d'informations de lumière ambiante est rempli ;
ii. si le critère de validité est rempli, la détermination de la concentration de l'analyte dans le fluide corporel par l'évaluation d'au moins une image d'au moins une partie d'au moins une région de test de réactif (118) d'une bandelette de test optique (116) sur laquelle est appliqué un échantillon du fluide corporel, l'image étant capturée par la caméra (114) du dispositif mobile (112) ;
dans lequel le procédé comprend en outre :
iii. si le critère de validité n'est pas rempli, la prévention automatique de la détermination de la concentration de l'analyte dans le fluide corporel.

2. Procédé selon la revendication précédente, dans lequel l'étape i.2 comprend la comparaison de l'au moins un élément d'informations de lumière ambiante avec au moins une valeur seuil.

3. Procédé selon la revendication précédente, dans lequel le critère de validité est rempli si l'une des conditions suivantes est remplie : l'au moins un élément d'informations de lumière ambiante a une valeur inférieure à la valeur seuil ; au moins un élément d'informations de lumière ambiante a une valeur ne dépassant pas la valeur seuil.

4. Procédé selon l'une quelconque des revendications précédentes, dans lequel l'étape iii. comprend la prévention d'au moins l'une parmi : la capture de l'image de la région de test de réactif (118) ; la demande à un utilisateur de positionner la bandelette de test optique (116) à l'intérieur d'un champ de vision de la caméra (114) ; la demande à un utilisateur d'appliquer l'échantillon du fluide corporel sur la région de test de réactif (118) de la bandelette de test optique (116) ; la piqûre d'un doigt de l'utilisateur pour appliquer l'échantillon du fluide corporel sur la région de test de réactif (118) de la bandelette de test optique (116) ; l'évaluation d'une réaction de formation de couleur de la région de test de réactif (118).

5. Procédé selon l'une quelconque des revendications précédentes, dans lequel l'étape ii. comprend :
ii.1 la capture de l'au moins une image de l'au moins une partie de l'au moins une région de test de réactif (118) sur laquelle est appliqué l'échantillon du fluide corporel ;
ii.2 la détermination d'au moins un élément d'informations optiques indiquant une réaction de formation de couleur de la région de test de réactif (118) ; et
ii.3 la déduction de la concentration de l'analyte en utilisant l'élément d'informations optiques.

6. Procédé selon la revendication précédente, l'étape ii. comprenant en outre au moins l'une parmi :
ii.4 l'invitation d'un utilisateur à appliquer l'échantillon du fluide corporel sur la région de test de réactif (118) ;
ii.5 l'invitation d'un utilisateur, avant de réaliser l'étape ii.1, à positionner la bandelette de test optique (116) à l'intérieur d'un champ de vision de la caméra (114) ;
ii.6 l'invitation d'un utilisateur, avant de réaliser l'étape ii.1, à positionner au moins une carte de référence de couleur (120) à l'intérieur d'un champ de vision de la caméra (114), la carte de référence de couleur (120) comprenant une pluralité de champs de référence de couleur (142) ayant des valeurs de couleur de référence connues.

7. Procédé selon l'une quelconque des revendications précédentes, dans lequel la caméra (114) et le détecteur de lumière ambiante (122) sont situés sur des côtés opposés du dispositif mobile (112).

8. Procédé selon l'une quelconque des revendications précédentes, dans lequel le capteur de lumière ambiante (124) est compris dans au moins un écran (132) du dispositif mobile (112).

9. Procédé selon l'une quelconque des revendications précédentes, dans lequel la caméra supplémentaire (126) comprend au moins une caméra avant (134) située sur un côté avant (130) du dispositif mobile (112), le côté avant (130) du dispositif mobile (112) comprenant au moins un élément de fonctionnement (136).

10. Procédé selon l'une quelconque des revendications précédentes, dans lequel le procédé est mis en œuvre par ordinateur, dans lequel la mise en œuvre par ordinateur comprend une application logicielle s'exécutant sur le dispositif mobile (112), dans lequel l'application logicielle comprend, après le démarrage de l'application logicielle, le guidage d'un utilisateur à travers une séquence de mesure (176), dans lequel la séquence de mesure (176) comprend au moins l'étape ii., dans lequel l'étape i. est réalisée totalement ou partiellement avant et/ou pendant la séquence de mesure (176).

11. Dispositif mobile (112) ayant au moins une caméra (114), le dispositif mobile (112) comprenant en outre au moins un détecteur de lumière ambiante (122), le détecteur de lumière ambiante (122) comprenant au moins un parmi un capteur de lumière ambiante (124) et une caméra supplémentaire (126), dans lequel le dispositif mobile (112) est configuré pour réaliser le procédé selon l'une quelconque des revendications précédentes.

12. Kit (110), comprenant le dispositif mobile (112) selon la revendication précédente, le kit (110) comprenant en outre au moins une bandelette de test optique (116) ayant au moins une région de test de réactif (118).

13. Programme informatique comprenant des instructions qui, lorsque le programme est exécuté par le dispositif mobile (112) selon l'une quelconque des revendications précédentes se référant à un dispositif mobile (112), amènent le dispositif mobile (112) à réaliser le procédé selon l'une quelconque des revendications précédentes se référant à un procédé.

14. Support de stockage lisible par ordinateur comprenant des instructions qui, lorsqu'elles sont exécutées par le dispositif mobile (112) selon l'une quelconque des revendications précédentes se référant à un dispositif mobile (112), amènent le dispositif mobile (112) à réaliser le procédé selon l'une quelconque des revendications précédentes se référant à un procédé.
